# EUROPÄISCHE PATENTSCHRIFT

(11) **EP 1 446 075 B1**
(45) Veröffentlichungstag und Bekanntmachung des Hinweises auf die Patenterteilung: **20.09.2006**
(21) Anmeldenummer: 02785294.6
(22) Anmeldetag: 24.10.2002
(51) Int. Cl.: A61F 2/36

(54) **SCHENKELHALSENDOPROTHESE FÜR EIN KÜNSTLICHES HÜFTGELENK**
FEMORAL NECK ENDOPROSTHESIS FOR AN ARTIFICIAL HIP JOINT
ENDOPROTHESE DE COL DU FEMUR POUR UNE ARTICULATION DE HANCHE ARTIFICIELLE

(30) Priorität: 22.11.2001 DE 10158558
(43) Veröffentlichungstag der Anmeldung: 18.08.2004
(73) Patentinhaber: ESKA Implants GmbH & Co., 23556 Lübeck (DE)
(72) Erfinder: GRUNDEI, Hans, 23558 Lübeck (DE)
(74) Vertreter: Fuchs Mehler Weiss & Fritzsche
(86) Internationale Anmeldenummer: PCT/EP2002/011891
(87) Internationale Veröffentlichungsnummer: WO 2003/043544

(56) Entgegenhaltungen:
- EP-A- 0 634 154
- EP-A- 0 679 375
- EP-A- 0 791 342
- EP-A- 1 205 163
- WO-A-01/82841
- WO-A-89/11837
- DE-A- 2 724 234
- DE-A- 3 334 058
- DE-A- 19 841 612
- DE-C- 19 610 741
- DE-C- 19 720 493
- DE-U- 20 207 913
- DE-U- 29 921 577
- FR-A- 2 626 169
- FR-A- 2 674 122
- US-A- 5 755 793

## Beschreibung

Es wird eine Schenkelhalsendoprothese für ein künstliches Hüftgelenk beschrieben. Der nächstliegende stand der Technik ist WO-A-0182841.

Zur Behandlung eines distruierten, d.h. zerstörten Hüftgelenkkopf, woraus erhebliche Schmerzempfindungen und gravierende funktionelle Einschränkungen des Gelenks resultieren, haben sich im wesentlichen zwei Therapiemöglichkeiten während der letzten Jahrzehnte durchgesetzt:

Zum einen ist es möglich, den Patienten mit einer Orthese zu versorgen, d.h. also mit einem externen Stützapparat. Neben der Tatsache, daß diese Versorgungsmöglichkeit kosmetisch völlig unakzeptable ist, ist sie auch versorgungstechnisch nur suboptimal.

Als weitere, weitverbreitete Möglichkeit ist es vorgesehen, den Patienten mit einer Endoprothese zu versorgen, namentlich mit einer Hüftstielendoprothese, bei welcher ein Stiel in den zuvor freigeräumten Knochenmarksraum des Femurs eingesetzt wird und dort zementlos oder unter Verwendung eines Zements fixiert wird. An der proximalen Seite weist eine derartige Endoprothese dann die Möglichkeit des Anschlusses einer künstlichen Gelenkkugel auf.

Gerade die letzte Möglichkeit ist stark in der Patentliteratur vertreten. An dieser Stelle sei nur beispielsweise auf die DE 94 12 408 U1 hingewiesen.

Unter den einen oder anderen der in der Patentliteratur vorgeschlagenen Hüftstielendoprothesen gibt es einige Ansätze, die recht vielversprechend sind im Hinblick auf die Langzeitstabilitiät im Patientenkörper. Früher oder später jedoch, beispielweise nach 10 bis 15 Jahren, ist zu beobachten, daß die Endoprothese einem Verschleiß unterworfen ist, welcher zu der Notwendigkeit führt, in einem Revisionseingriff die implantierte Endoprothese zu entfernen und durch eine neue zu ersetzen. Dies ist freilich nicht ganz unproblematisch, da durch die Resektion und Ausräumung des Knochenmarkraumes ein nicht unerheblicher Anteil natürlichen Knochenmaterials bei der Erstimplantation entfernt worden ist. Das einmal entfernte Material fehlt dann unter Umständen bei einem Revisionseingriff. Dies kommt insbesondere dann zum Tragen, wenn die Patienten relativ jung sind, bei denen also von vornherein davon auszugehen ist, daß sie im Laufe ihres Lebens mindestens einem Revisionseingriff unterworfen werden müssen.

Eine Endoprothese, die mit einer geringfügigeren Resektion natürlichen Knochenmaterials auskommt als die bekannten Hüftstielendoprothesen ist bekannt geworden aus der DE 27 24 234 C2. Das darin beschriebene Prothesenteil wird im oberen Bereich eines Femurs, aber unterhalb des Trochanter minors implantiert, ohne daß ein Stiel in den Knochenmarksraum reichen würde. Diese auch als Druckscheibenprothese bezeichnete Prothese greift im wesentlichen mit einer proximalen Druckplatte über die Kortikalis des resezierten Femurs im Bereich des entfernten Hüftgelenkkopfes. Sie wird mit einer Druckplatte, die lateral am Femur anliegt, verspannt, derart, daß alle mechanischen Kräfte zwischen der Endoprothese und dem Femur direkt in die Kortikalschicht des Femurs eingeleitet werden, wodurch eine als unzulässig empfundene mechanische Beanspruchung der Spongiosa vermieden werden soll. Lediglich eine geringe Menge von Knochenzement ist für die Arretierung der Druckscheibe im proximalen Bereich notwendig.

Der Philosophie dieser Prothese liegt - wie ausgeführt - die Annahme zugrunde, daß die Spongiosa des Femurs möglichst wenig beansprucht werden soll. Dies wird erkauft durch eine extreme Belastung der Kortikalis des Femurs, indem nämlich sämtliche Kräfte darauf abgelastet und darin eingeleitet werden. Dies entspricht nach heutiger Erkenntnis in keiner Weise den natürlichen Belastungsverhältnissen.

Eine ganz ähnlich wirkende Prothese zeigt im übrigen die WO 89/11837, bei der die Funktion der proximalen Druckplatte gemäß der vorerwähnten Druckschrift wahrgenommen wird durch den speziell ausgebildeten Hüftgelenkkopf, der innenseitig mit einer Ausnehmung versehen ist, an der sich innenseitig der reserzierte Femurstumpf unter Druckerzeugung anlegt.

Zwei Prothesen, bei denen augenscheinlich die Hauptlast von der Spongiosa des Femurknochens aufgenommen werden soll, sind bekannt aus der FR 26 26 169 A1 und FR 26 74 122 A1. Bei der erstgenannten Druckschrift der beiden ist vorgesehen, einen proximal einen Steckkonus aufweisenden Gewindebolzen in die Spongiosa einzuschrauben. Dies dürfte innerhalb kürzester Zeit zu schweren Instabilitätsproblem führen. Bei der zweitgenannten Druckschrift der beiden wird eine einen Steckkonus tragende Platte mittels einer Reihe von Knochenschrauben befestigt, wobei die Knochenschrauben in die Spongiosa reichen. Auch hieraus ergeben sich ernsthafte Stabilitätsfragen, da die Spongiosa von Natur aus im Verhältnis zur Kortikalis des Femurknochens punktuell nur gering belastbar ist.

Einen anderen Weg beschreitet die DE-A-196 01 340. In dieser Druckschrift wird eine Schenkelhalsendoprothese beschrieben, die eine zementlos im oberen Bereich eines Femurs unterhalb des Trochanter majors implantierbare Hülse aufweist, die an ihrem proximalen Ende mit einer künstlichen Gelenkkugel versehen ist. Ihre Hülsenaußenseite ist zumindest teilweise mit einer offenmaschigen dreidimensionalen Raumnetzstruktur belegt. Darüber hinaus ist eine Zugplatte vorgesehen, an der eine Zugschraube anschlagbar ist, die durch eine Durchbohrung im distalen Ende der Hülse in deren Inneren setzbar und mit einem dort versehenen Gewinde verschraubbar ist.

Zur Vorbereitung der Implantation wird zunächst der zerstörte Femurkopf durch eine subcapitale Resektion entfernt, und zwar im wesentlichen orthograd zur Schenkelhalsachse. Sodann wird mittels eines Fräsers, der eine im wesentlichen gleiche Außenkontur wie die zu implantierende Hülse aufweist, in den Schenkelhals getrieben und eine entsprechende Bohrung oder Aushöhlung in der Spongiosa des Femurs hergestellt. Sodann kann die Hülse in dem ausgefrästen Raum im Schenkelhals gesetzt werden. Zur lateralen Festlegung der Hülse ist vorgesehen, von außen an dem Femur die erwähnte Zugplatte anzulegen, welche mittels einer Zugschraube mit dem Inneren der Hülse verschraubbar ist. Zu diesem Zweck muß die Kortikalis lateral aufgebohrt werden, so daß die Zugschraube durch die Kortikalis in das Innere der Hülse geführt werden kann, um dort verschraubt zu werden. Die Zugplatte bildet mit der Zugschraube ein im Prinzip bekanntes System der Zuggurtung.

Im Gegensatz zu langstieligen Hüftlangstielendoprothesen findet die Endoprothese der vorerwähnten Art in der Metaphyse des Femurknochens Platz. Gegenüber den Langstielendoprothesen sind nur relativ geringe Resektionen von natürlichem Knochenmaterial notwendig, weswegen sie sich den natürlichen Verhältnissen erheblich besser anpaßt als jene der vorerwähnten Druckschriften. Hierzu trägt auch bei, daß die Hülse und das Hülsenende zumindest teilweise mit der erwähnten offenmaschigen dreidimensionalen Raumnetzstruktur belegt ist, in welche Knochentrapekel einwachsen und so für eine dauerhafte Fixation der Endoprothese in der Metaphyse sorgen.

Nun gibt es Indikationen, bei denen die Implantation der Endoprothese gemäß der vorerwähnten Druckschrift wenig erfolgsversprechend ist. Dies ist insbesondere dann der Fall, wenn aufgrund anderer Komplikationen nicht mit einem hinreichenden Einwachsen von Knochentrapekeln in die dreidimensionale Raumnetzstruktur zu rechnen ist. Stoffwechselstörungen wie Diabetes seien als nur ein Beispiel genannt. Gleichwohl besteht das Bedürfnis, auch die hiervon betroffenen Patienten mit einer Schenkelhalsendoprothese zu versorgen, um auch ihnen den Vorteil der geringstmöglichen Resektionen bieten zu können.

Darüber hinaus kann die Einleitung von Belastungskräften in die Kortikalis durch die Zugschraube in einigen Fällen problematisch sein.

Vor diesem Hintergrund ist es nun die Aufgabe der vorliegenden Erfindung, eine Schenkelhalsendoprothese der vorerwähnten Art so weiterzubilden, daß sie in der Metaphyse des Femurs fixiert werden kann, ohne auf die an sich bewährte Fixationsmöglichkeit durch Einwachsen von Knochenmaterial in die Oberfläche des Implantates zurückzugreifen. Hierbei gilt es zu berücksichtigen, daß grundsätzlich Ausfräsungen im natürlichen Knochen, in welche das Implantat einzementiert werden soll, größer ist als das Implantat selbst, damit dieses von einem Zementmantel in einer Stärke von mindestens 2 mm umgeben werden kann. Das Aushärten des Zementes dauert üblicherweise ca. 10 min., während der das Implantat seine Stellung noch verändern kann. Dies bedeutet ein gewisses Risiko dafür, daß das Implantat nach Aushärten des Zementes eine nicht gewünschte Stellung einnimmt, welche zu Fehlfunktionen des Gelenkes führen können.

Gelöst wird diese Aufgabe durch eine Schenkelhalsendoprothese für ein künstliches Hüftgelenk mit den Merkmalen des Anspruchs 1. Weitere vorteilhafte Ausführungsformen sind in den Unteransprüchen angegeben.

Demgemäß weist die Schenkelhalsendoprothese auf:
- eine im oberen Bereich eines Femurs unterhalb des Trochanter majors implantierbare Hülse, die an ihrem proximalen Ende einen Kragen zur Anlage an Resektionsflächen des Schenkelhalses aufweist und mit deren proximalem Ende ein Adapter in Form eines Doppelkonus zur Aufnahme einer Gelenkkugel verbindbar ist, wobei
- die Hülse von ihrem proximalen Ende zu ihrem distalen Ende konisch sich verjüngend ausgebildet ist und ballig verrundet oder flach ausgebildet ist,
- der Doppelkonus des Adapters zwei Steckkonen, deren Längsachsen sich in einem Winkel α im Bereich von O < α < 30° schneiden, sowie an der Basis des Steckkonus, der mit der Hülse verbindbar ist, eine Markierung aufweist, welche die Drehposition des Steckkonus in der Hülse anhand einer an dem Kragen vorgesehenen Skala anzeigt.

Die erfindungsgemäße Schenkelhalsendoprothese wird also mit Zement im Schenkelhals implantiert. Wie schon erwähnt, sind Ausfräsungen in einem Röhrenknochen, in dem eine Endoprothese implantiert werden soll, größer als die Endoprothese selbst. Nach der Ausfräsung bestehen also Freiheitsgrade für die Lage des Implantates im Knochen. Dies tritt bei den zementlos zu implantierenden Implantaten nicht auf. Die Freiheitsgrade führen dazu, daß das Implantat in der einen oder anderen Lage fixiert werden kann. Hierbei spielt eine große Erfahrung des Operateurs eine wesentliche Rolle. Das Implantat wird von einem Zementmantel mit einer Mindestdicke von 2 mm umgeben. Entsprechend groß muß die Ausfräsung sein. Es ist einsichtig, daß nach direktem Einsetzen des Implantates und Hinzugabe des Knochenzementes die Lage des Implantates immer noch veränderlich ist, da das Aushärten des Zementes ca. 10 min. dauert. Hier muß also u. U. eine Korrekturmaßnahme erfolgen, die erfindungsgemäß darin besteht, den Steckkonus in der Hülse zu verdrehen. Dies geschieht kontrolliert anhand der Markierung an der Basis des in der Hülse sitzenden Steckkonus sowie der Skala am Kragen. Mittels Verdrehung des Doppelkonus in der Hülse vermag der Operateur etwaige Fehllagen des Implantates in der Metaphyse des Femurs auszugleichen, so daß das künstliche Hüftgelenk später die volle Funktionsbreite inne hat, ohne Beschwerden seitens des Patienten hervorzurufen. Die konische Ausbildung der Endoprothese entspricht der mehr oder weniger konischen Ausbildung des Schenkelhalses, stellt also eine anatomische Adaption dar. Darüber hinaus ist die Kräfteverteilung in der Metaphyse günstiger. Das ballige Ende erleichtert das Einführen in die Ausfräsung im Schenkelhals.

Gemäß einer weiteren bevorzugten Ausführungsform ist vorgesehen, daß die Hülse einen rotationsunsymmetrischen Querschnitt aufweist. Dies bedeutet eine zusätzliche Sicherheit gegenüber einem Verdrehen der Endoprothese in der Metaphyse des Femurs und damit eine Sicherheit gegen etwaige Fehlstellungen des künstlichen Hüftgelenkes.

Die Rotationsunsymmetrie kann bevorzugt dadurch realisiert werden, daß der Querschnitt der Hülse ovalär ist.

In diesem Falle ist die Hauptachse des ovalären Hülsenquerschnitts besonders bevorzugt dorsal-ventral orientiert.

Alternativ kann ein Hülsenquerschnitt trapezförmig oder im wesentlichen dreieckig ausgebildet sein, wobei dann in vorteilhafter Weise die Ecken jeweils verrundet sind.

Die Erfindung wird nachfolgend anhand eines Ausführungsbeispieles näher erläutert.

Hierbei zeigt:
- Figur 1: die Ansicht des Implantates mit aufgesetzter Gelenkkugel,
- Figur 2: ein Implantat gemäß Figur 1 mit dem Doppelkonus in anderer Drehposition,
- Figur 3: das Implantat ohne Adapter,
- Figur 4: eine Schnittansicht entlang der Linie IV-IV in Figur 1,
- Figur 5: eine Ansicht in Richtung des Pfeiles V in Figur 3 gemäß einer anderen Ausführungsform,
- Figur 6: die Ansicht des Doppelkonus in Richtung des Pfeiles VI in Figur 2, und
- Figur 7: eine Ansicht des Implantates in Richtung des Pfeiles VII in Figur 3.

Nachfolgend bezeichnen gleiche Bezugszeichen dieselben Teile.

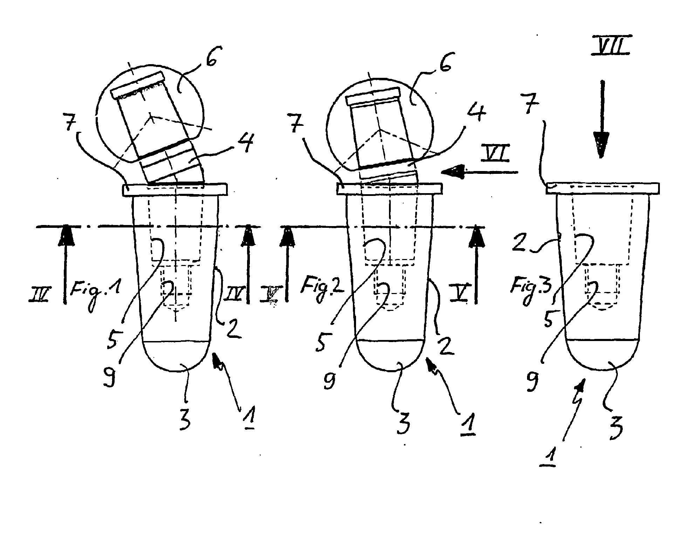

In Figur 1 ist die Schenkelhalsendoprothese allgemein mit dem Bezugszeichen 1 versehen. Diese besteht im wesentlichen aus einer Hülse 2 mit ihrem balligen Ende 3. Im Inneren der Hülse 2 ist eine konische Steckhülse 5 ausgeformt, in die ein Steckkonus eines Adapters 4 in Doppelkonusform gesteckt werden kann. Auf dem weiteren Steckkonus des Adapters 4 sitzt eine künstliche Gelenkkugel 6, die dann mit einer künstlichen Beckenpfanne des künstlichen Hüftgelenks zusammenarbeitet.

Proximal ist an die Hülse 2 ein Kragen 7 angeformt, der über den Außendurchmesser der Hülse 2 hinausragt. Der Kragen 7 dient zur Anlage an der Resektionsfläche des Schenkelhalses und einer Begrenzung der Einführtiefe der Endoprothese 1 in den Schenkelhals.

Am Boden der Steckhülse 5 ist eine Gewindebohrung 9 eingelassen. Diese dient dazu, im Revisionsfall ein Werkzeug aufzunehmen, mittels dessen Hilfe die Schenkelhalsendoprothese 1 aus dem Schenkelhals herausgezogen werden kann.

Zur Korrektur einer eventuellen Fehllage der Endoprothese 1 im Schenkelhals des Femurs ist der Adapter 4 mit wenigstens einer Markierung 11 versehen, die im vorliegenden Falle durch zwei weitere Markierungen 11a und 11b ergänzt worden ist. Die Markierung 11 repräsentiert gewissermaßen die Nullage, wohingegen die Markierungen 11a und 11b Auslenkungen um beispielsweise 5° repräsentieren.

Diese Markierungen 11, 11a und 11b arbeiten zusammen mit einer Skala 12 (Figur 7), welche am Kragen 7 vorgesehen ist. Die Skala besteht aus mehreren Markierungen, von welchen jede eine Auslenkung der Drehposition des Adapters 4 aus der Position O° um jeweils 10° repräsentiert.

Zunächst wird der Operateur den Adapter 4 noch relativ lose in die Steckhülse 5 stecken, und zwar so, daß die Markierung 11 mit der Skalenmarkierung O° der Skala 12 fluchtet. Sodann wird das Implantat in die Ausfräsung im Schenkelhals gesetzt und der Knochenzement wird in den Zwischenraum zwischen Implantat und Spongiosa gespritzt. In dieser Phase kann der Operateur etwaige Fehlstellungen der Hülse 2 im Schenkelhals dadurch kompensieren, daß er den noch nicht fest in der Steckhülse 5 sitzenden Adapter 4 in die eine oder andere Richtung dreht. Dies ist beim Übergang von Figur 1 zu Figur 2 veranschaulicht. Hier ist der Adapter 4 um einige Grad verdreht worden, so daß sich die Stellung der Gelenkkugel 6 deutlich verändert hat. Dementsprechend ändert sich die Lage der Markierungen 11, 11a und 11b gegenüber den Einteilungen auf der Skala 12.

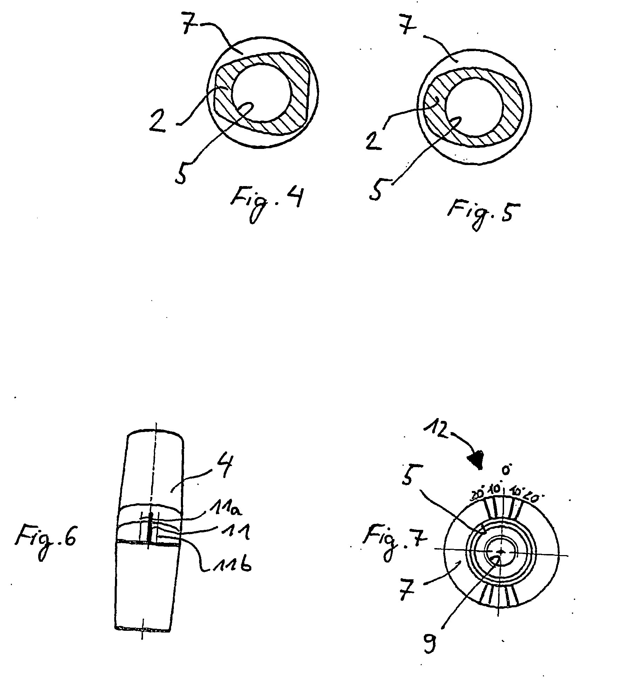

In den Figuren 4 und 5 sind zwei unterschiedliche Ausführungsformen für die Kontur der Hülse 2 abgebildet. Die Figur 4 zeigt eine Schnittansicht entlang der Schnittlinie IV-IV in Figur 1 und Figur 5 eine Ansicht entlang der Schnittlinie V-V in Figur 2.

Figur 4 zeigt einen fast trapezförmigen Querschnitt der Hülse 2. Figur 5 hingegen zeigt eine fast ovaläre Querschnittsfläche der Hülse 2. Hintergrund dieser unterschiedlichen Ausbildungen ist es, die Hülse 2 rotationsunsymmetrisch zu gestalten, damit eine Lageveränderung der Hülse 2 in der Metaphyse des Femurs sicher unterbunden werden kann.

### Bezugszeichen

- 1: Schenkelhalsendoprothese
- 2: Hülse
- 3: Ende
- 4: Adapter in Doppelkonusform
- 5: konische Steckhülse
- 6: künstliche Gelenkkugel
- 7: Kragen
- 9: Gewindebohrung am Boden der Steckhülse
- 11a, 11b: Markierungen auf Doppelkonus
- 12: Skala auf Kragen

## Patentansprüche

1. Schenkelhalsendoprothese (1) für ein künstliches Hüftgelenk, aufweisend
- eine im oberen Bereich eines Femurs unterhalb des Trochanter majors implantierbare Hülse (2), die an ihrem proximalen Ende einen Kragen (7) zur Anlage an Resektionsflächen des Schenkelhalses aufweist und mit deren proximalem Ende ein Adapter (4) in Form eines Doppelkonus zur Aufnahme einer Gelenkkugel (6) verbindbar ist, wobei
- die Hülse von ihrem proximalen Ende zu ihrem distalen Ende konisch sich verjüngend ausgebildet ist und ein balliges Ende (3) aufweist,
- **dadurch gekennzeichnet, daß** der Doppelkonus des Adapters (4) zwei Steckkonen aufweist, deren Längsachsen sich in einem Winkel im Bereich von O < α < 30° schneiden, sowie an der Basis des Steckkonus, der mit der Hülse (2) verbindbar ist, eine Markierung (11, 11a, 11b) aufweist, welche die Drehposition des Steckkonus in der Hülse (2) anhand einer an dem Kragen (7) vorgesehenen Skala (12) anzeigt.

2. Schenkelhalsendoprothese nach Anspruch 1, bei der die Hülse (2) einen rotationssymmetrischen Querschnitt aufweist.

3. Schenkelhalsendoprothese nach Anspruch 2, bei der der Querschnitt der Hülse (2) ovalär ist.

4. Schenkelhalsendoprothese nach Anspruch 3, bei der die Hauptachse des ovalären Hülsenquerschnitts dorsal-ventral orientiert ist.

5. Schenkelhalsendoprothese nach Anspruch 2, bei der der Querschnitt der Hülse (2) trapezförmig ist.

6. Schenkelhalsendoprothese nach Anspruch 2, bei der der Querschnitt der Hülse (2) im wesentlichen dreieckig ist.

7. Schenkelhalsendoprothese nach Anspruch 5 oder 6, bei der die Ecken des Querschnitts verrundet sind.

## Claims

1. Femoral neck endoprosthesis (1) for an artificial hip joint, having
- a sleeve (2) which can be implanted in the upper region of a femur below the trochanter major, and which has at its proximal end a collar (7) for resting on resection surfaces of the femoral neck and an adapter (4) in the form of a double cone can be connected to its proximal end to receive a joint head (6), wherein
- the sleeve is designed to be conically tapering from its proximal end to its distal end and has a spherical end (3), **characterised in that**
- the double cone of the adapter (4) has two plug-in cones, the longitudinal axes of which intersect at an angle in the range from 0 < α < 30°, and on the base of the plug-in cone, which can be connected to the sleeve (2), has a marking (11, 11a, 11b), which indicates the position of rotation of the plug-in cone in the sleeve (2) using a scale (12) provided on the collar (7).

2. Femoral neck endoprosthesis according to claim 1, in which the sleeve (2) has a rotationally symmetrical cross-section.

3. Femoral neck endoprosthesis according to claim 2, in which the cross-section of the sleeve (2) is oval.

4. Femoral neck endoprosthesis according to claim 3, in which the main axis of the oval sleeve cross-section is orientated dorsally-ventrally.

5. Femoral neck endoprosthesis according to claim 2, in which the cross-section of the sleeve (2) is trapezoidal.

6. Femoral neck endoprosthesis according to claim 2, in which the cross-section of the sleeve (2) is essentially triangular.

7. Femoral neck endoprosthesis according to claim 5 or 6, in which the corners of the cross-section are rounded.

## Revendications

1. Endoprothèse de col du fémur (1) pour une articulation artificielle de hanche comportant un manchon (2) implantable dans la région supérieure d'un fémur au-dessous du grand trochanter, ledit manchon comportant à son extrémité proximale un collet (7) pour l'appui contre les surfaces de résection du col du fémur, et l'extrémité proximale dudit manchon pouvant être reliée à un adaptateur (4) affectant la forme d'un double cône afin de recevoir une bille d'articulation (6), étant précisé que le manchon s'effile de façon conique depuis son extrémité proximale jusqu'à son extrémité distale et comporte une extrémité bombée (3), **caractérisée en ce que** le double cône de l'adaptateur (4) comporte deux cônes enfichables dont les axes longitudinaux se coupent en un angle situé dans une plage de 0 < α < 30°, et comporte à la base du cône enfichable, lequel peut être relié au manchon (2), un marquage (11, 11a, 11b) lequel à l'aide d'une graduation (12) prévue au niveau du collet (7), indique la position de rotation du cône enfichable dans le manchon (2).

2. Endoprothèse de col du fémur selon la revendication 1, dans laquelle le manchon (2) comporte une section transversale à symétrie de révolution.

3. Endoprothèse de col du fémur selon la revendication 2, dans laquelle la section transversale du manchon (2) est ovalaire.

4. Endoprothèse de col: du fémur selon la revendication 3, dans laquelle l'axe principal de la section transversale ovalaire du manchon est orienté de façon dorso-ventrale.

5. Endoprothèse de col du fémur selon la revendication 2, dans laquelle la section transversale du manchon (2) est trapézoïdale.

6. Endoprothèse de col du fémur selon la revendication 2, dans laquelle la section transversale du manchon (2) est essentiellement triangulaire.

7. Endoprothèse de col du fémur selon la revendication 5 ou 6, dans laquelle les angles de la section transversale sont arrondis.
